# EUROPEAN PATENT APPLICATION

(11) **EP 4 481 382 A1**
(43) Date of publication of application: **25.12.2024**
(21) Application number: 23756366.3
(22) Date of filing: 14.02.2023
(51) Int. Cl.: G01N 33/493

(54) **EXCRETED URINARY MATTER AMOUNT AND URINE AMOUNT EVALUATION METHOD**

(30) Priority: 16.02.2022 JP 2022022473
(71) Applicant: Tokyo University of Pharmacy & Life Sciences, Hachioji-shi, Tokyo 192-0392 (JP); Nakayama, Akiyoshi, Tokorozawa-shi, Saitama 359-8513 (JP); Matsuo, Hirotaka, Tokorozawa-shi, Saitama 359-8513 (JP)
(72) Inventor: KIMIYOSHI, Ichida, Tokyo, 192-0392 (JP); HIROTAKA, Matsuo, Tokorozawa-shi Saitama 359-8513 (JP); AKIYOSHI, Nakayama, Tokorozawa-shi Saitama 359-8513 (JP)
(74) Representative: Michalski Hüttermann & Partner Patentanwälte mbB
(86) International application number: PCT/JP2023/005003
(87) International publication number: WO 2023/157841

(57) **Abstract**

[Problem] An object of the present invention is to provide a method of estimating/evaluating a urinary excreted substance amount per unit time based on a finding that a urinary substance concentration can be quite accurately and practically estimated by considering an estimated glomerular filtration rate and a net urinary substance excretion rate.

[Solution] The method of evaluating a urinary substance excretion amount per unit time includes: multiplying a concentration of an arbitrary urinary excreted substance present in the serum by: an estimated glomerular filtration rate per unit time, a numerical value (net urinary substance excretion rate) obtained by dividing a clearance of the arbitrary urinary excreted substance present in the serum by a glomerular filtration rate, and a predetermined coefficient.

## Description

### Technical Field

The present invention relates to a method of estimating a urinary excreted substance amount per unit time, a method of estimating/evaluating a urine volume per unit time, and a method of estimating/evaluating a urine concentration rate.

### Background Art

In the prior art, a substance excretion amount was estimated by dividing a urinary substance concentration by a urinary creatinine concentration (Non Patent Literatures 1, 2, and 3). This estimation of the substance excretion amount is used to classify the severity of chronic kidney disease (CKD) of a patient in an example of urinary albumin or urinary protein. In addition, with respect to "gCr-corrected Na", it is shown that a daily urinary Na excretion amount is predicted by dividing urinary Na by creatinine, that a daily Na intake is estimated, and that the estimated daily Na intake is used as a resource for life guidance (guidance on salt reduction) of a hypertensive patient. However, this method was inaccurate.

### Citation List

### Non Patent Literature

Non Patent Literature 1: Ginsberg, J. M., Chang, B. S., Matarese, R. A. & Garella, S. Use of single voided urine samples to estimate quantitative proteinuria. N. Engl. J. Med. 309, 1543-1546, doi:10.1056/NEJM198312223092503 (1983).
Non Patent Literature 2: Evidence-based CKD Clinical Practice Guideline 2018 (https://cdn.jsn.or.jp/data/CKD2018.pdf)
Non Patent Literature 3: Main text 26 of GUIDELINES FOR THE MANAGEMENT OF HYPERTENSION 2019 (http://www.jpnsh.jp/data/jsh2019/JSH2019_noprint.pdf)

### Summary of Invention

### Technical Problem

In the prior art, based on an observation study (Non Patent Literature 1) reporting that "a daily urinary creatinine excretion amount is 1 g/day/1.73 m^2", a substance excretion amount was estimated by dividing a urinary substance concentration by a urinary creatinine concentration (Non Patent Literatures 2 and 3). In the example of urinary albumin or urinary protein, this estimation of the substance excretion amount is used to predict the daily urinary albumin excretion amount by dividing a urinary albumin concentration or a urinary protein concentration by the urinary creatinine concentration as a "urinary albumin/Cr ratio" or a "urinary protein/Cr ratio" and to classify the severity of chronic kidney disease (CKD) of the patient. In addition, in an example of urinary sodium, as the "gCr-corrected Na", it is shown that a daily urinary sodium excretion amount is predicted by dividing a urinary sodium concentration by a urinary creatinine concentration, that a daily sodium intake is estimated, and that the estimated daily sodium intake is used as a resource for life guidance (guidance on salt reduction) of a hypertensive patient. However, it is known that the urinary creatinine excretion amount is greatly affected by the body type such as a muscle amount, exercise, and diet, and there is a problem that it is difficult to adapt the premise that "the daily urinary creatinine excretion amount is 1 g/day/1.73 m^2" (Non Patent Literature 1) to all humans, and it is actually inaccurate.

An object of the present invention is to provide a method of estimating/evaluating a urinary excreted substance amount per unit time, a method of estimating/evaluating a urine volume per 24 hours, and a method of estimating/evaluating a urine concentration rate, based on a finding that a urinary substance concentration can be quite accurately and practically estimated by considering an estimated glomerular filtration rate and a net urinary substance excretion rate.

### Solution to Problem

A method of estimating/evaluating a urinary excreted substance amount per unit time according to the present invention is characterized by including: multiplying a concentration of an arbitrary urinary excreted substance present in the serum by: an estimated glomerular filtration rate per unit time, a numerical value (net urinary substance excretion rate) obtained by dividing a clearance of the arbitrary urinary excreted substance present in the serum by a glomerular filtration rate, and a predetermined coefficient.

The urinary excreted substance amount per unit time may be selected from uric acid, sodium, magnesium, calcium, inorganic phosphoric acid, potassium, albumin, proteins, sugars including glucose, oxalic acid, citric acid, amino acids, uremic toxin substances, drugs, toxic substances, substances that can be measured by urine biochemical analysis, and substances that can be detected by urine metabolome analysis.

A method of estimating/evaluating a urinary excreted substance amount per unit time according to the present invention includes: multiplying a concentration of creatinine present in the serum by: an estimated glomerular filtration rate per unit time, a concentration ratio between the urinary excreted substance and urinary creatinine, and a predetermined coefficient including a numerical value (net creatinine excretion rate) obtained by dividing a creatinine clearance by a glomerular filtration rate.

It may be characterized in that 1.44 calculated from 60 × 24/100 as the predetermined coefficient and 1.40 (when creatinine is measured by an enzyme method) or 1.16 (when creatinine is measured by a Jaffe method), which is a net urinary creatinine excretion rate, or any coefficient of net urinary creatinine excretion rates corrected by other creatinine concentration measurement methods are multiplied. The content can be rephrased as "net urinary substance excretion rate" = "concentration ratio between the urinary excreted substance and urinary creatinine" × "numerical value (net creatinine excretion rate) obtained by dividing creatinine clearance by glomerular filtration rate".

The method of evaluating/evaluating a urinary excreted substance amount per unit time may be characterized in that the unit time is 24 hours.

Provided is a method of estimating/evaluating a urine volume per unit time (e.g., per 24 hours) by dividing, by a urinary excreted substance concentration, the urinary excreted substance amount per unit time (e.g., per 24 hours) estimated/evaluated by the above-described method of estimating/evaluating a urinary excreted substance amount per unit time.

Provided is a method of estimating/evaluating a urine concentration rate using the above-described estimated urinary excreted substance amount per arbitrary unit time or 24 hours, the method including: estimating/evaluating a urine concentration rate based on a numerical value obtained by dividing an estimated glomerular filtration rate by an estimated urine volume per arbitrary unit time or 24 hours.

Provided are a method of evaluating a urinary excreted substance amount per unit time, a method of estimating/evaluating a urine volume per 24 hours, and a method of estimating/evaluating a urine concentration rate, characterized by: measuring, by a measuring instrument installed in a toilet bowl or the like, concentrations, in the urine, of creatinine and a urinary excreta selected from uric acid, sodium, magnesium, calcium, inorganic phosphoric acid, potassium, albumin, proteins, sugars including glucose, oxalic acid, citric acid, amino acids, uremic toxin substances, drugs, toxic substances, substances that can be measured by urine biochemical analysis, and substances that can be detected by urine metabolome analysis.

Provided are a method of estimating/evaluating a urinary excreted substance amount per unit time, a method of estimating/evaluating a urine volume per 24 hours, and a method of estimating/evaluating a urine concentration rate, characterized by: collecting urine with a urine collecting device or collecting instrument installed in a toilet bowl or the like; and measuring concentrations, in the urine, of creatinine and a urinary excreta selected from a urinary excreta selected from uric acid, sodium, magnesium, calcium, inorganic phosphoric acid, potassium, albumin, proteins, sugars including glucose, oxalic acid, citric acid, amino acids, uremic toxin substances, drugs, toxic substances, substances that can be measured by urine biochemical analysis, and substances that can be detected by urine metabolome analysis.

### Advantageous Effects of Invention

The present invention has made it possible to quite accurately and practically estimate a urinary substance concentration without requiring urine accumulation by considering the estimated glomerular filtration rate and the net urinary substance excretion rate. In addition, the present invention has made it possible to estimate the estimation/evaluation of a urine volume per arbitrary unit time or 24 hours, and to estimate/evaluate a urine concentration rate.

### Brief Description of Drawings

Fig. 1 is Correspondence (1) between estimated value and actually measured value.
Fig. 2 is Examination (1) on error between estimated value and actually measured value.
Fig. 3-1 is Correspondence (2) between estimated value and actually measured value.
Fig. 3-2 is Correspondence (2) between estimated value and actually measured value.
Fig. 4 is Examination (2) on error between estimated value and actually measured value.

### Best Mode for Carrying Out the Invention

An Example in which a urinary uric acid excretion amount is estimated based on creatinine (measurement by an enzymatic method) will be described.

As a result of actual measurement of "serum creatinine value", "estimated glomerular filtration rate", "urinary uric acid concentration", "urinary creatinine concentration" and "body surface area" of a subject A, they were "0.9 mg/dl", "100 ml/min/1.73 m^2", "30 mg/dl", "60 mg/dl" and "1.80", respectively.

When these values were inserted into the present invention for calculation, the urinary excreted substance amount per unit time was 14.4*1.40*(30/60)*0.9*100 = 907.2 mg/day. The urine volume was 14.4*1.4*0.9*100/60*100*1.80/1.73 = 3146.96 mg/day. The urine concentration rate was (1/1.40)*(60/0.9) = 47.62 times. In this way, it was possible to estimate/evaluate the urinary excreted substance amount, the urine volume, and the urine concentration rate per 24 hours, respectively.

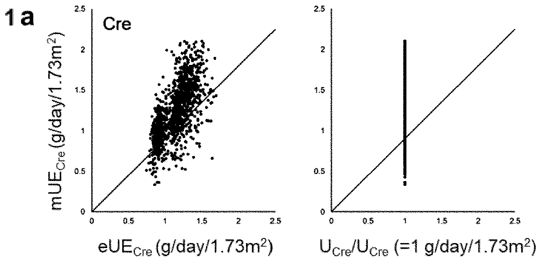

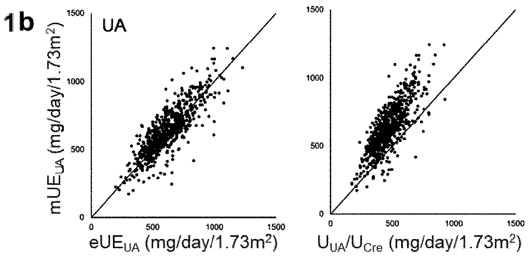

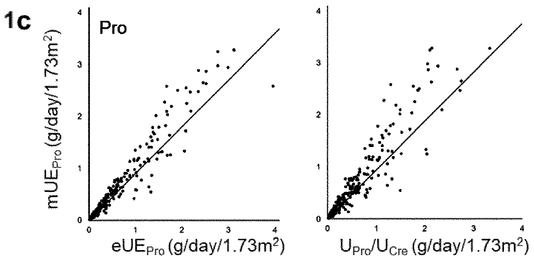

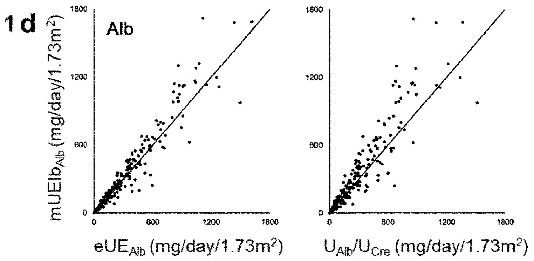

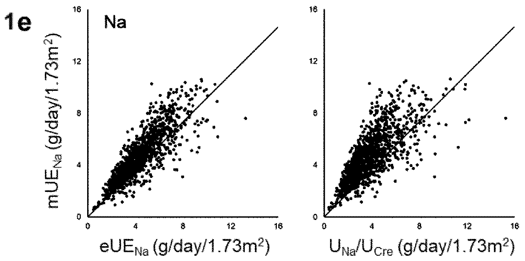

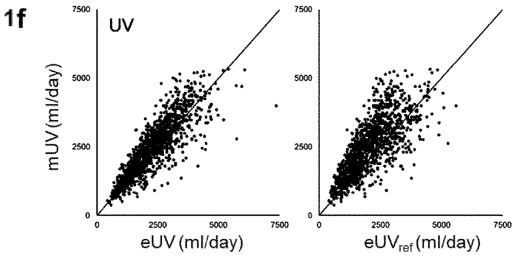

Next, it will be explained that the estimation method according to the present invention corresponds to an actually measured value. Figs. 1 to 4 show analysis results of 1,238 Japanese people. The correspondence to the actually measured values can demonstrate the usefulness of the estimation method according to the present invention. Fig. 1 shows correspondence (1) between estimated values and actually measured values. Estimation (horizontal axis) and actual measurement (vertical axis) were compared in a Japanese population for each of urinary substances: (a) creatinine (Cre), (b) uric acid (UA), (c) protein (Pro), (d) albumin (Alb), (e) sodium (Na), and (f) urine volume (UV). Each left panel is based on the present estimation equation, and each right panel is based on a conventional method of division by creatinine, and the left one is along the line of y=x as compared with the right one (along an ideal line where the estimated values and the actually measured values match).

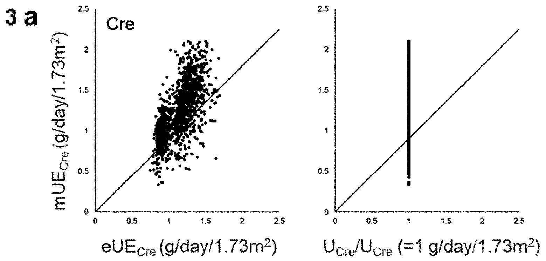

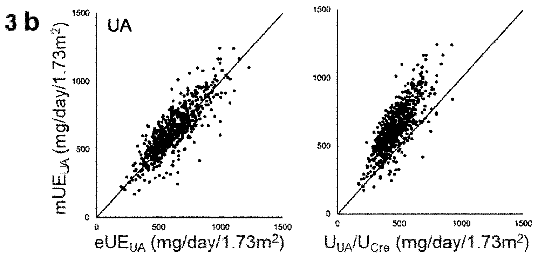

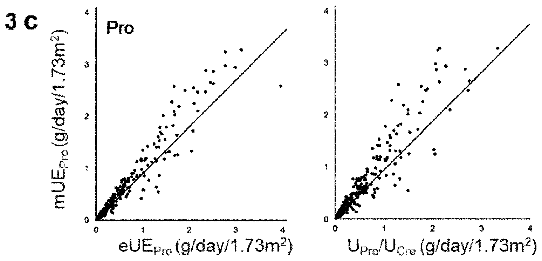

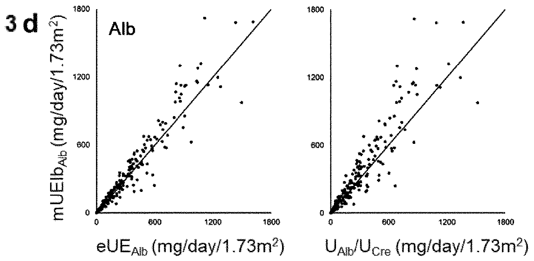

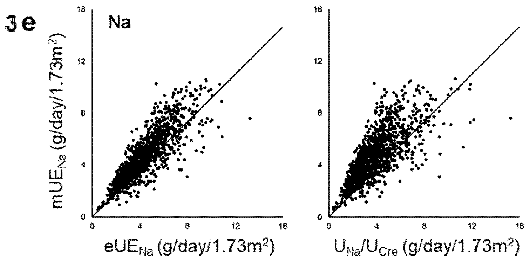

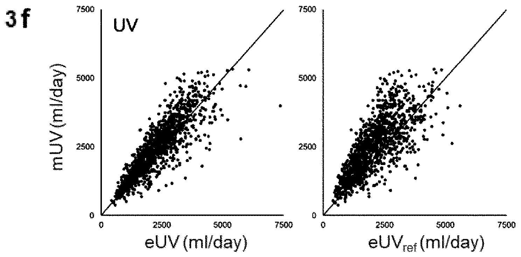

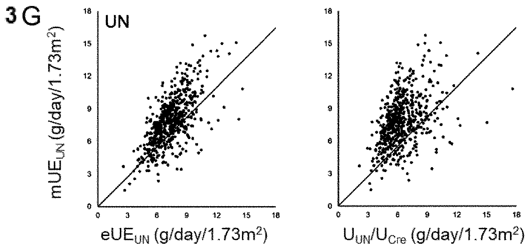

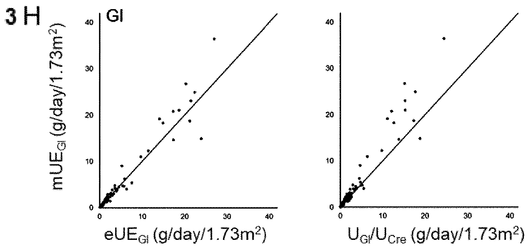

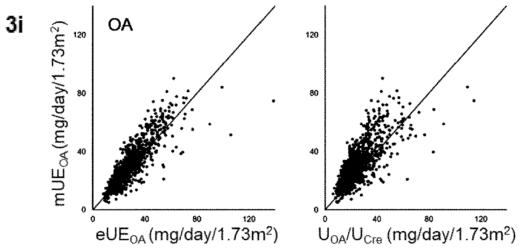

Fig. 3 illustrates comparisons between estimated values and actually measured values in terms of various errors: root mean squared error (RMSE), Bias (estimated values - actually measured values), Precision (absolute values of estimated values - actually measured values), and Accuracy (percentage of the estimated values falling within ±20% (P20) or ±30% (P30) of the actually measured values) for the various urinary substances in Fig. 1. For all of RMSE, Bias, and Precision, it is found that the results obtained using the present estimation equation show a smaller error and a higher accuracy than those obtained by the conventional method of division by creatinine.

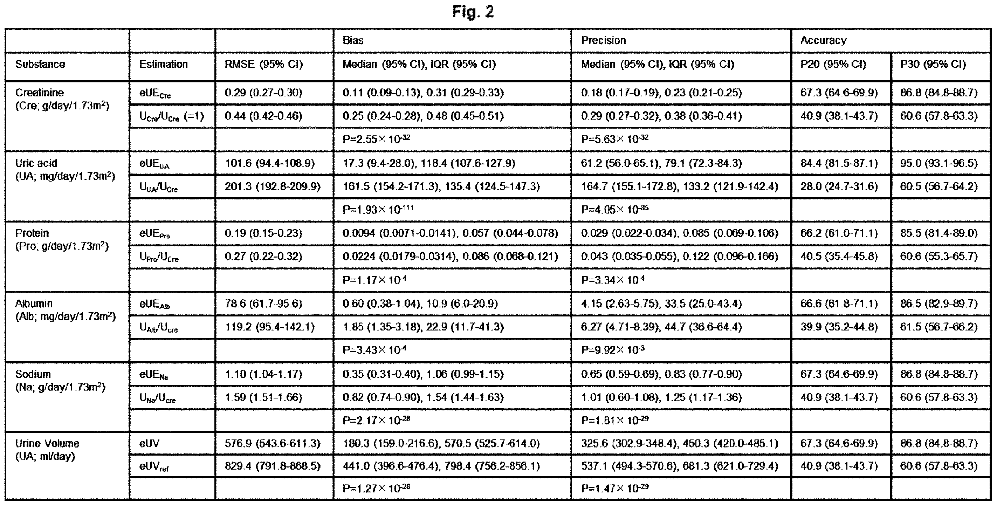

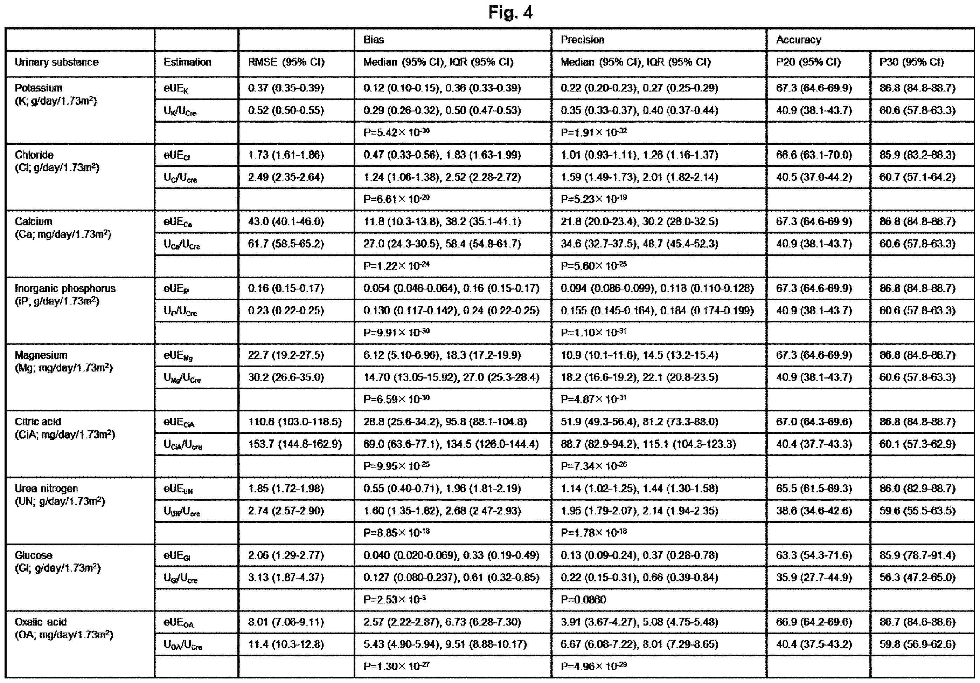

Fig. 4 shows examination (2) on errors between estimated values and actually measured values. For the various urinary substances in Fig. 2, comparisons were made between estimated values and actually measured values in terms of various errors: root mean squared error (RMSE), Bias (estimated values - actually measured values), Precision (absolute values of estimated values - actually measured values), and Accuracy (percentage of the estimated values falling within ±20% (P20) or ±30% (P30) of the actually measured values) (Fig. 4). For all of RMSE, Bias, and Precision, it is found that the results obtained using the present estimation equation show a smaller error and a higher accuracy than those obtained by the conventional method of division by creatinine.

Based on the above-described implementation data in Figs. 1 to 4, the estimation method according to the present invention has usefulness and a remarkable effect as compared with the conventional invention.

The method of estimating a urine volume according to the present invention can be used to evaluate a drinking water amount, and to evaluate whether or not a subject is in a dehydrated state. The method of estimating a urine concentration rate according to the present invention can be used to evaluate the function of reabsorption/secretion of a urinary excreted substance in a kidney tubule (proximal tubule, distal tubule, descending limb/ascending limb of Henle, or collecting tubule) and a collecting duct.

### Industrial Applicability

The present invention contributes to an effective diagnostic technique, since it is possible to estimate the urinary excreted substance amount, the urine volume, and the urine concentration rate per four hours by actually measuring some measurement items of serum and urine. In addition, some measurement items of urine are applied to toilet bowls that are used on a daily basis, so that the present invention can provide a toilet bowl that enables health management and present an index of a lifestyle for health management, and thus is industrially useful.

## Claims

1. A method of evaluating a urinary substance excretion amount per unit time, the method being a method of estimating a urinary excreted substance amount per unit time, comprising:
multiplying a concentration of an arbitrary urinary excreted substance present in the serum by:
an estimated glomerular filtration rate per unit time,
a numerical value (net urinary substance excretion rate) obtained by dividing a clearance of the arbitrary urinary excreted substance present in the serum by a glomerular filtration rate, and
a predetermined coefficient.

2. A method of evaluating a urinary substance excretion amount per unit time, the method being a method of estimating a urinary excreted substance amount per unit time, comprising:
multiplying a concentration of creatinine present in the serum by:
an estimated glomerular filtration rate per unit time,
a concentration ratio between the urinary excreted substance and urinary creatinine, and
a predetermined coefficient including a numerical value (net creatinine excretion rate) obtained by dividing a creatinine clearance by a glomerular filtration rate.

3. The method of evaluating a urinary excreted substance amount per unit time according to claim 1 or 2, wherein
1.44 calculated from 60 × 24/100 as the predetermined coefficient and
1.40 (when creatinine is measured by an enzyme method) or 1.16 (when creatinine is measured by a Jaffe method), which is a net urinary creatinine excretion rate, or any coefficient of net urinary creatinine excretion rates corrected by other creatinine concentration measurement methods are multiplied.

4. The method of evaluating a urinary excreted substance amount per unit time according to any one of claims 1 to 3, wherein
the unit time is 24 hours.

5. A method of evaluating a urine volume per unit time by dividing an estimated urinary excreted substance amount per arbitrary unit time by a urinary excreted substance concentration
by the method according to any one of claims 1 to 4.

6. The method of evaluating a urinary excreted substance amount per unit time according to any one of claims 1 to 5, wherein
the urinary excreted substance amount per unit time is
selected from uric acid, sodium, magnesium, calcium, inorganic phosphoric acid, potassium, albumin, proteins, sugars including glucose, oxalic acid, citric acid, amino acids, uremic toxin substances, drugs, toxic substances, substances that can be measured by urine biochemical analysis, and substances that can be detected by urine metabolome analysis.

7. A method of estimating a urine concentration rate
using the estimated urinary excreted substance amount per unit time according to claim 5, wherein
a urine concentration rate is evaluated
based on a numerical value obtained by dividing an estimated glomerular filtration rate by an estimated urine volume per unit time.

8. The method according to any one of claims 1 to 7, further comprising:
measuring, by a measuring instrument installed in a toilet bowl or the like, concentrations, in the urine, of creatinine and a urinary excreta selected from uric acid, sodium, magnesium, calcium, inorganic phosphoric acid, potassium, albumin, proteins, sugars including glucose, oxalic acid, citric acid, amino acids, uremic toxin substances, drugs, toxic substances, substances that can be measured by urine biochemical analysis, and substances that can be detected by urine metabolome analysis.

9. The method according to any one of claims 1 to 7, further comprising: collecting urine with a urine collecting device or collecting instrument installed in a toilet bowl or the like; and measuring concentrations, in the urine, of creatinine and a urinary excreta selected from uric acid, sodium, magnesium, calcium, inorganic phosphoric acid, potassium, albumin, proteins, sugars including glucose, oxalic acid, citric acid, amino acids, uremic toxin substances, drugs, toxic substances, substances that can be measured by urine biochemical analysis, and substances that can be detected by urine metabolome analysis.
